(19) Europäisches Patentamt

European Patent Office

Office européen des brevets

(11) **EP 0 618 426 B1**

(12) **EUROPÄISCHE PATENTSCHRIFT**

(45) Veröffentlichungstag und Bekanntmachung des Hinweises auf die Patenterteilung:
**04.12.2002 Patentblatt 2002/49**

(51) Int Cl.⁷: **G01F 11/02**, A61M 5/145

(21) Anmeldenummer: **94104671.6**

(22) Anmeldetag: **24.03.1994**

(54) **System zur Dosierung von Flüssigkeiten**

System for dosing of liquids

Système pour le dosage des liquides

(84) Benannte Vertragsstaaten:
**DE ES FR GB IT NL SE**

(30) Priorität: **02.04.1993 DE 4310808**

(43) Veröffentlichungstag der Anmeldung:
**05.10.1994 Patentblatt 1994/40**

(73) Patentinhaber: **Roche Diagnostics GmbH**
**68298 Mannheim (DE)**

(72) Erfinder: **Sattler, Stephan**
**D-82380 Peissenberg (DE)**

(56) Entgegenhaltungen:
WO-A-88/10383          DE-A- 4 020 522
US-A- 4 838 857         US-A- 4 978 335

**Beschreibung**

Die vorliegende Erfindung betrifft ein System zur Dosierung von Flüssigkeiten, das eine Dosiervorrichtung, eine Vorrichtung zur Bewegung des Kolbens der Dosiervorrichtung, eine Vorrichtung zur Eingabe von Daten und eine elektronische Einheit zur Berechnung eines Dosierhubes beinhaltet. Außerdem betrifft die Erfindung ein Verfahren zur Dosierung mit dem vorgenannten System und eine in dem System verwendbare Dosiervorrichtung.

Zur exakten Dosierung von Flüssigkeiten in Analysengeräten waren bisher Systeme bekannt, die sehr kostenaufwendige Dosiervorrichtungen beinhalten. Im Stand der Technik sind für Dosierungen mit geringeren Genauigkeitsanforderungen Vorrichtungen üblich, die einem Kolbenprober entsprechen. Die folgende Anordnung ist allgemein bei Spritzen bekannt, sie soll daher nachfolgend als Spritzenkörper bezeichnet werden. Ein Zylinder ist einseitig mit einer Wandung verschlossen, die eine im Vergleich zum Durchmesser des Zylinders kleine Öffnung aufweist, durch die Flüssigkeiten austreten können. Die kleine Öffnung kann in einen weiteren Zylinder einmünden, welcher an der der Öffnung abgewandten Seite konisch verengt sein kann. Innerhalb des Zylinders ist ein dichtender Kolben beweglich. In dem durch Kolben und Zylinder gebildeten Hohlraum befindet sich die zu dosierende Flüssigkeit. Durch eine Bewegung des Kolbens kann Flüssigkeit zur Dosierung herausgedrückt werden. Eine solche Dosiervorrichtung wird allgemein als Spritze bezeichnet.

Ein gebräuchliches Material für Dosiervorrichtungen vom Typ eines Kolbenprobers sind Gläser. Diese Materialien besitzen den Vorteil, daß sie chemisch und biologisch weitgehend inert sind, so daß gebräuchliche Flüssigkeiten zur Verwendung in Dosiervorrichtungen innerhalb der Dosiervorrichtung gelagert werden können, ohne daß Reaktionen zwischen Flüssigkeit und Material der Vorrichtung stattfinden. Dosiervorrichtungen aus Glas besitzen außerdem den Vorteil, daß sie thermisch sterilisiert werden können, was besonders für ihre Verwendung in klinischen Analysensystemen von Bedeutung ist. Die Herstellung gläserner Dosiervorrichtungen erfolgt aus langen Glasröhren. Diese werden zunächst in kleinere Röhren zerlegt, die wiederum an jeweils einem Ende so eingeschmolzen werden, daß eine kleine Öffnung verbleibt. Der im Zylinder bewegliche Kolben kann ebenfalls aus Glas gefertigt sein, was jedoch relativ aufwendig ist, da der Kolben so geschliffen werden muß, daß er zwar im Zylinder beweglich ist, jedoch den Austritt von Flüssigkeit durch den von Kolben und Zylinder gebildeten Zwischenraum verhindert. Für den Kolben werden daher bevorzugt Materialien verwendet, die auch bei üblichen Fertigungstoleranzen den Anforderungen an Beweglichkeit und Dichtigkeit genügen. In der Regel werden Kolben aus Gummi oder Kunststoffen gefertigt. Aufgrund des Herstellungsprozesses weisen Dosiervorrichtungen aus Glas auch in der Serienproduktion einen schwankenden Innendurchmesser auf. Beispielsweise schwankt selbst der Innendurchmesser vorsortierter Spritzen um 0.1 mm bei einem mittleren Innendurchmesser von etwa 10 mm. Der durch diese Schwankung hervorgerufene Fehler in der Volumenbestimmung kann mit der Fehlerrechnung ermittelt werden:

$$V = h \cdot \pi \cdot r^2$$

$$dV = h \cdot \pi \cdot 2 \cdot r \cdot dr$$

$$\frac{\Delta V}{V_0} \cdot 100\,\% = 2 \cdot \frac{\Delta r}{r_0} \cdot 100\,\%$$

V: Volumen
h: Dosierhub
r: Radius des Zylinders
$\Delta V$: Volumenfehler
$\Delta r$: Fehler des Radius

Für die obengenannten Daten ergibt sich damit ein Fehler in der Volumenbestimmung von etwa 2 %. Für viele Dosierzwecke, besonders als Teilschritte einer Analyse, ist eine Volumenbestimmung mit einem Fehler > 1 % inakzeptabel.

Bekannt sind im Stand der Technik ebenfalls Dosiervorrichtungen aus Kunststoffen. Die Herstellung einer Dosiervorrichtung aus Kunststoff ist hinreichend bekannt. Wird der äußere Zylinder der Dosiervorrichtung durch Einpressen in eine Form hergestellt, so können Durchmesserschwankungen weitgehend vermieden werden. Der Fehler in der Volumendosierung kann ohne zusätzlichen Aufwand jedoch nicht unter 0.5 % gebracht werden. Dosiervorrichtungen aus Kunststoff besitzen den Nachteil, daß in Abhängigkeit vom Material mehr oder minder starke Deformationen auftreten, wenn die Vorrichtung mechanisch belastet wird, wie z. B. bei einem Dosiervorgang selbst. Die Verwendung von Dosiervorrichtungen aus Kunststoffen besitzt auch den Nachteil, daß der Kontakt der zu dosierenden Flüssigkeit mit dem Kunststoff in vielen Fällen zu unerwünschten Reaktionen führt. In der Praxis ist man daher bestrebt, die Kontaktzeit von Flüssigkeit und Dosiervorrichtung möglichst kurz zu halten. Es sind Systeme bekannt, bei denen Flüssigkeit aus einem Glasgefäß durch eine Kunststoff-Dosiervorrichtung angesaugt wird, um kurz darauf dosiert in ein weiteres Gefäß, z. B. ein Analysengefäß, abgegeben zu werden.

Für Analysen, bei denen Dosierungsfehler unter 0.5 % gesenkt werden sollen, sind sogenannte Präzisions-Diluter gebräuchlich. Diese Art von Dosiervorrichtung entspricht in ihrer Funktionsweise ebenfalls dem Prinzip des Kolbenprobers, sie ist jedoch aufgrund ihres aufwendigen Herstellungsprozesses wesentlich teurer als die bereits beschriebenen Vorrichtungen. Die Eigenschaften eines Präzisions-Diluters machen es unmöglich, diesen Typ der Dosiervorrichtung zum Transport von Flüssigkeiten zu verwenden.

In der WO 88/10383 wird ein System zur Dosierung von Flüssigkeiten beschrieben, bei dem über einen Motor der Kolben eines in dem System befindlichen Spritzenkörpers bewegt wird, um Flüssigkeit aus dem Spritzenkörper abzugeben. Bei der beschriebenen Vorrichtung wird über einen Hebelmechanismus der Außendurchmesser des in dem System befindlichen Spritzenkörpers abgetastet und auf Basis des gemessenen Wertes erkannt, welcher Typ von Spritzenkörper vorliegt. Auf Basis dieses Wertes werden die zur Dosierung erforderlichen Kenndaten aus einer Tabelle ausgelesen und unter Berücksichtigung von vom Benutzer eingegebenen Daten über ein zu dosierendes Volumen die Dosierung ausgeführt. Das Dosiersystem der WO 88/10383 kann mit verschiedenen Typen von Standardspritzenkörpern arbeiten und ist insoweit offen, daß es auch eine Dosierung mit nicht Standardspritzenkörpern nach entsprechender Umprogrammierung ermöglicht.

Aufgrund der Nachteile bekannter Dosiervorrichtungen war es die Aufgabe der Erfindung, ein neues System zur Verfügung zu stellen, das präzise Dosierungen mit einer preisgünstigen und einfacheren Vorrichtung ermöglicht.

Die Aufgabe wurde durch ein System zur Dosierung von Flüssigkeiten gelöst, das eine Dosiervorrichtung, eine Vorrichtung zur Bewegung des Kolbens der Dosiervorrichtung, ein Lesegerät zum Einlesen von Daten und eine elektronische Einheit zur Berechnung eines Dosierhubes besitzt, wobei das System einen Datenträger beinhaltet, der Daten , die für den Innendurchmesser der individuellen Dosiervorrichtung charakteristisch sind, speichert. Außerdem wurde ein Verfahren zur präzisen Dosierung gefunden.

Ein wesentlicher Bestandteil des erfindungsgemäßen Systems zur Dosierung ist eine geeignete Dosiervorrichtung. Zur Erfindung gehört demnach eine Dosiervorrichtung, die eine Flüssigkeit enthält, und die dadurch gekennzeichnet ist, daß für ihren Innendurchmesser spezifische Daten auf der Dosiervorrichtung angebracht sind. Die Vorrichtung kann erfindungsgemäß einer bereits beschriebenen Anordnung nach dem Prinzip des Kolbenprobers entsprechen. Es kommen somit in erster Linie die als Kolbenprober und Spritze bezeichneten Anordnungen in Frage. Spritzenkörper aus Glas werden beispielsweise von der Firma Münnerstädter Glaswarenfabrik hergestellt. Es können Spritzenkörper mit Angabe der gewünschten Maße direkt bestellt werden. Ebenfalls die verwendeten Gläser können aus einer breiten Palette ausgewählt werden. Bevorzugt sind solche Gläser, die chemisch weitgehend inert sind und somit auch eine Lagerung von Flüssigkeiten zur Dosierung in der Dosiervorrichtung ermöglichen. Die Schwankungen des Innendurchmessers betragen auch bei vorsortierten Lieferungen noch etwa 0.1 mm. Spritzenkörper aus Kunststoff können beispielsweise von den Firmen Treff oder Eppendorff bezogen werden. Im Handel erhältliche Dosiervorrichtungen aus Kunststoff, auch Dispenser genannt, besitzen eine Genauigkeit von 0,6 % bis 1,5 %.

Obwohl die Durchmesserschwankungen verschiedener Spritzenkörper relativ groß sind, besitzt beobachtungsgemäß jeder Spritzenkörper einen über seine Länge nahezu konstanten Innendurchmesser. Wird dieser zum individuellen Spritzenkörper gehörende Innendurchmesser (z. B. in Millimetern) bestimmt und in einer Art und Weise gespeichert, bei der eine Zuordnung von Daten und Spritzenkörper gegeben ist, so kann ein Dosierhub berechnet werden, der eine präzise Dosierung ermöglicht. Für Dosierungsvorrichtungen mit einer bevorzugt zylindrischen Gestalt ergibt sich der Dosierhub (h) aus dem zu dosierenden Volumen (V) und dem individuellen Innendurchmesser (d) der Dosiervorrichtung:

$$h = \frac{4 \cdot V}{\pi \cdot d^2}$$

Allgemein ergibt sich für Dosiervorrichtungen mit beliebigem, jedoch konstantem Querschnitt:

$$h = \frac{V}{A}$$

h: Dosierhub
V: zu dosierendes Volumen
A: Querschnittsfläche der Dosiervorrichtung

Mit einer Dosiervorrichtung kann eine einzelne Dosierung durchgeführt werden, bevorzugt ist jedoch die Verwendung einer Dosiervorrichtung zur mehrfachen Dosierung. Dies kann entweder dadurch erreicht werden, daß mehrfach Flüssigkeit in eine erfindungsgemäße Dosiervorrichtung eingezogen und dosierend abgegeben wird. Bevorzugt wird es erreicht, indem aus einer gefüllten Dosiervorrichtung mehrfach Flüssigkeit dosierend abgegeben wird, ohne daß zwischen den Flüssigkeitsabgaben neue Flüssigkeit in die Dosiervorrichtung eingezogen wird.

Bei einer Serie von Dosiervorrichtungen ist es ebenfalls möglich, die auftretenden Innendurchmesser in Gruppen aufzuteilen und statt eines Innendurchmessers, eine für die Gruppe der jeweiligen Dosiervorrichtung charakteristische Kennung oder den gemeinsamen Innendurchmesser zu speichern.

Die Bestimmung des Innendurchmessers beschriebener Vorrichtungen kann mit einer Zahl von Verfahren erfolgen, z. B. mechanisch mit einer Mikrometerschraube oder optisch mit Methoden der Bildverarbeitung. Bei einem bevorzugten Verfahren wird der Innendurchmesser durch eine dem Fachmann bekannte Anordnung aus Düse und Prallplatte gemessen.

Der ermittelte Innendurchmesser kann auf verschiedene Arten gespeichert werden, z. B. in einem Computer, auf Papier oder auf einem Magnetstreifen. Bevorzugt ist jedoch die Speicherung in Form eines Balkencodes. Die gespeicherten Daten können weitere für die Analyse relevante Informationen enthalten, z. B. Fassungsvermögen der Dosiervorrichtung oder bei gefüllten Dosiervorrichtungen z. B. Art, Menge und Konzentration der zu dosierenden Flüssigkeit. Die Zuordnung von Dosiervorrichtung und Datenmaterial ist bevorzugt dadurch gegeben, daß der Datenträger direkt an der Dosiervorrichtung angebracht ist, z. B. in Form eines Balkencodes, der sich auf dem Spritzenkörper befindet. Es ist jedoch auch möglich, daß sich die Informationen auf der Verpackung der Dosiervorrichtung befinden. Eine weitere Möglichkeit der Zuordnung ist gegeben, wenn sich Dosiervorrichtungen in einer ersten Anordnung befinden, die Daten in einer zweiten Anordnung vorliegen und eine Vorschrift existiert, die eine eindeutige Zuordnung von Elementen der beiden Anordnungen beschreibt.

Zur Erfindung gehören ebenfalls Systeme zur Dosierung, bei denen der Innendurchmesser einer Dosiervorrichtung mit einem der bereits genannten Verfahren innerhalb des Systems gemessen wird. Das Meßergebnis kann gespeichert und in gleicher Weise wie eingelesene Angaben über den Innendurchmesser zur Durchführung einer Dosierung verwendet werden.

Innerhalb des Spritzenkörpers einer erfindungsgemäßen Dosiervorrichtung befindet sich ein Kolben, der an den Innenwandungen des Spritzenkörpers dichtend anliegt. Dieser Kolben kann z. B. aus Glas, Keramik oder Metallen gefertigt sein, bevorzugt sind jedoch Materialien, welche eine Deformierbarkeit besitzen, die einerseits eine Abdichtung gewährleistet, andererseits jedoch Dosierungsfehler durch Verformung des Kolbens, z. B. beim Dosierprozeß, weitgehend vermeiden. Bevorzugte Materialien sind Kunststoffe, z. B. Polyethylen, Polypropylen, Gummi, Silikongummi, auch Teflon und synthetische Plaste.

Die Kolben und Spritzenkörper beinhaltende Dosiervorrichtung kann entweder leer oder bevorzugt gefüllt mit zu dosierender Flüssigkeit in das System zur Dosierung eingebracht werden. Wird eine außerhalb des Systems gefüllte Dosiervorrichtung verwendet, so ist sie bevorzugt versiegelt. Eine Spritze kann beispielsweise durch eine Kappe auf der Kanüle versiegelt sein. Bevorzugt sind Spritzen aus Glas, deren Austrittsöffnung versiegelt ist. Die Versiegelung kann beispielsweise aus einer dünnen Metallfolie oder aus Kunststoffen gefertigt sein. Besonders bevorzugt ist eine Versiegelung in Form eines Septums, das z. B. mit Aluminiumblech auf die Austrittsöffnung einer Spritze aufgeklemmt ist. Das Septum kann die im Stand der Technik als für Septen geeignet bekannten Materialien, wie z. B. Kunststoffe und Plaste, beinhalten.

Sofern in der Dosiervorrichtung Flüssigkeiten enthalten sind, so sind dies bevorzugt wässrige Lösungen und besonders bevorzugt Reagenzlösungen zur Durchführung einer Analyse. Eine Reagenzlösung kann z. B. Enzyme, Farbstoffe oder farbstoffbildende Substanzen, Säuren, Laugen, Puffer und andere Hilfsstoffe enthalten. Reagenzlösungen besitzen die Eigenschaft, sich mit einer oder mehreren nachzuweisenden Substanzen in spezifischer Weise so umzusetzen, daß ein detektierbares Signal entsteht.

Bei dem erfindungsgemäßen System wird die Dosiervorrichtung mit Hilfe eines Halters an einer Antriebsmechanik befestigt. Die für den Innendurchmesser der Dosiervorrichtung charakteristischen Daten können beispielsweise automatisch beim Einlegen der Dosiervorrichtung eingelesen werden, z. B. durch ein Strichcode-Lesegerät oder einen Magnetstreifenleser, oder können vom Benutzer des Systems, z. B. über eine Tastatur, eingegeben werden. Es ist vorteilhaft, wenn zusammen mit Daten für den Innendurchmesser ebenfalls Daten über Art, Menge und Konzentration des in der Dosiervorrichtung enthaltenen Materials an das System übergeben werden. Das System besitzt ebenfalls eine Mechanik, welche von einem Motor getrieben wird, die in der Lage ist, den Kolben der Dosiervorrichtung zu bewegen. Gebräuchliche Antriebsvorrichtungen für den Kolben der Dosiervorrichtung werden beispielsweise von der Firma Hamilton für Präzisions-Diluter vertrieben. Der Motor der Antriebsvorrichtung wird von einer Elektronik gesteuert, die aus Daten der Dosiervorrichtung, Daten des Inhaltes der Dosiervorrichtung und vom Benutzer des Systems eingegebenen Daten der durchzuführenden Dosierung die Ansteuerung des Motors berechnet. In einer bevorzugten Ausführungsform wird ein Schrittmotor verwendet, da bei diesen Motoren über Impulssequenzen eine exakt definierte Zahl von Voll- oder Teildrehungen der Motorwelle erreicht werden kann.

Das Einlesen von Daten für eine durchzuführende Dosierung kann beispielsweise über eine Tastatur, einen Codestreifenleser oder einen Magnetkartenleser erfolgen.

Die Erfindung umfaßt ebenfalls ein Verfahren zur Dosierung von Flüssigkeiten durch eine Dosiervorrichtung mit den Schritten

- Einlesen von für den Innendurchmesser der individuellen Dosiervorrichtung charakteristischen Daten

- Einlesen von für eine durchzuführende Dosierung spezifischen Daten

- Berechnung eines oder mehrerer Dosierhübe aus den eingelesenen Daten

- Ausführung eines oder mehrerer Dosierhübe.

Vorrichtungen für das Einlesen von Daten und der mögliche Inhalt der Daten wurden bereits geschildert. Die Berechnung eines Dosierhubes basiert auf diesen Daten. Zusätzlich gehen in die Berechnung Kenndaten des Systems ein, die z. B. das Verhältnis von Drehung der Motorwelle und resultierender Kolbenbewegung beinhalten. Die Ausführung eines Dosierhubes kann wie bereits beschrieben über einen Schrittmotor und eine Mechanik durchgeführt werden.

Zur Erfindung gehört ebenfalls ein Verfahren zur Analyse mit einem System zur Dosierung von Flüssigkeiten, das folgende Schritte beinhaltet:

- Einlesen von physikalischen Daten der individuellen Dosiervorrichtung

- Einlesen von Daten für den Innendurchmesser der individuellen Dosiervorrichtung charakteristischen Daten

- Einlesen von für eine durchzuführende Analyse spezifischen Daten

- Zugabe von Flüssigkeit aus einer Dosiervorrichtung zu einer Probe und Detektierung eines oder mehrerer Signale

- Auswertung der Analyse durch Zuordnung von aus der Dosiervorrichtung abgegebener Flüssigkeitsmenge und detektierten Signalen.

Ebenfalls gehört ein Verfahren zur Dosierung von Flüssigkeiten zur Erfindung, bei dem der Innendurchmesser der Dosiervorrichtung innerhalb des Systems gemessen wird.

Werden mit einer Dosiervorrichtung mehrere Analysen durchgeführt, so können Daten der Dosiervorrichtung und ihres Inhaltes gespeichert werden, brauchen also nicht vor jeder Analyse erneut eingelesen zu werden.

Besonders wichtig sind genaue Dosierungen bei der Durchführung von Analysen. Es sind hier zwei Typen von Analysen und damit auch Dosierverfahren zu unterscheiden. Bei einem ersten Analysentyp wird zu der Analysensubstanz in einer wässrigen Lösung von Stoffen, z. B. Körperflüssigkeiten wie Blut oder Harn, eine definierte Menge Reagenzlösung aus einer Dosiervorrichtung zugegeben. Diese Vorgehensweise kann z. B. bei enzymatischen Analysen angewandt werden. Es finden Umsetzungen von Analysenlösung und Reagenzlösung statt, die zu einem detektierbaren Signal, wie z. B. einer Farbänderung führen. Die Auswertung der Analyse erfolgt auf der Grundlage des detektierbaren Signals. Das für die Durchführung einer solchen Analyse notwendige Dosierverfahren entspricht im wesentlichen dem weiter oben beschriebenen Verfahren zur Dosierung.

Bei einem zweiten Typ von Analyse erfolgt eine Zugabe von Reagenzlösung zur Analysenlösung und die Detektierung eines Signals im wesentlichen gleichzeitig. Es ist mit einer Dosiervorrichtung erfindungsgemäßer Art möglich, Flüssigkeit aus der Dosiervorrichtung entweder in kleinen Portionen oder kontinuierlich zu der Analysenlösung zu geben. Es kann während dieser Zugabe beispielsweise die Wasserstoffionenkonzentration oder die Farbe der Lösung detektiert werden. Durch eine Zuordnung von detektiertem Signal und der aus der Dosiervorrichtung abgegebenen Flüssigkeitsmenge ist nach prinzipiell im Stand der Technik bekannten Verfahren eine Auswertung der Analyse möglich.

Ein Spezialfall des soeben beschriebenen Analysenverfahrens ist die Verwendung eines erfindungsgemäßen Systems analog zu einer Bürette. Beispielsweise könnte die Dosiervorrichtung so lange Reagenzflüssigkeit zu einer Analysenlösung geben, bis ein Benutzer eine Farbänderung erkennt und die Zugabe von Reagenzflüssigkeit stoppt. Das System zur Dosierung kann für den Benutzer die abgegebene Flüssigkeitsmenge z. B. in Millimetern auf einer Anzeigevorrichtung darstellen, mit der dieser die Analyse auswerten kann.

Die Erfindung besitzt gegenüber dem Stand der Technik den Vorteil, daß auch mit preisgünstigen Dosiervorrichtungen eine präzise Dosierung durch die Verwendung individueller Daten der Dosiervorrichtung ermöglicht wird. Die Möglichkeit, vorgefüllte Dosiervorrichtungen einzusetzen, die nach ihrer Benutzung verworfen werden, vermeidet nicht nur Probleme der Verschleppung, z. B. von Analysenlösung, sondern reduziert auch die Handhabung von Flüssigkeiten durch den Anwender auf ein Minimum.

Die Figuren 1 bis 4 zeigen spezielle Ausführungsformen eines erfindungsgemäßen Systems bzw. einzelner Elemente.

Figur 1: Dosiervorrichtung in Form einer Spritze

Figur 2: Dosiervorrichtung in Form einer Karpule (2a) und eine Anstechvorrichtung (2b)

Figur 3: System zur Dosierung von Flüssigkeiten

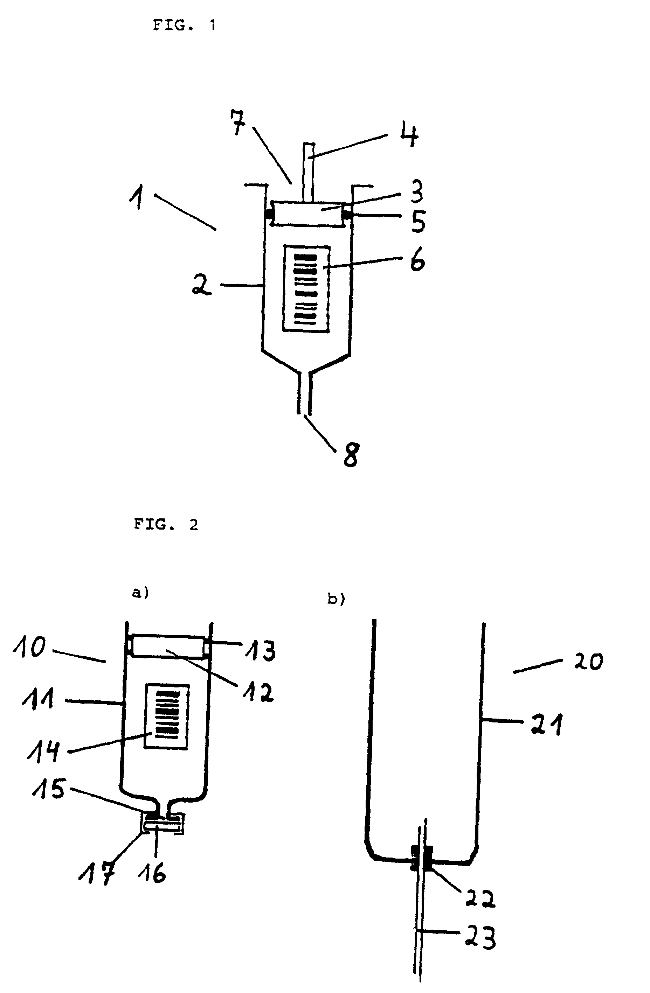

Figur 1 zeigt eine erfindungsgemäße Dosierungsvorrichtung (1). Der Spritzenmantel (2) besitzt eine zylindrische Form mit zwei Öffnungen. Die größere der beiden Öffnungen stellt die Kolbenöffnung (7) dar, durch sie kann ein Kolben (3) in den Spritzenmantel (2) eingeschoben werden. Durch die kleinere Öffnung kann Flüssigkeit aus der Dosiervorrichtung (1) abgegeben werden, sie wird daher Austrittsöffnung (8) genannt. Der Kolben (3) ist aus Glas gefertigt, um ihn ist ein 0-Ring (5) aus Gummi gelegt, der dafür sorgt, daß der Kolben (3) dichtend im Spritzenmantel (2) läuft. Eine Betätigung des Kolbens (3) erfolgt über einen Stempel (4). der Innendurchmesser des Spritzenmantels (2) ist als Strichcode (6) auf dem Spritzenmantel (2) angebracht.

In Figur 2 ist eine Karpule (10) dargestellt, die ebenfalls eine erfindungsgemäße Ausführungsform einer Dosiervorrichtung ist. Der Spritzenmantel (11) ist aus Glas gefertigt. In ihm befindet sich ein Kolben (12) aus Silikonkunststoff. Die Abdichtung wird in diesem Fall durch zwei Lippen (13) erreicht, die durch ringförmige Verdickungen gegeben sind, die den Kolben (12), der die Form eines flachen Zylinders besitzt, umgeben. Auf dem Spritzenmantel (11) ist ein Strichcode (14) aufgedruckt, der eine Kennung für den Innendurchmesser der Karpule (10), eine Bezeichnung der Flüssigkeit in der Karpule (10) und deren Konzentration beinhaltet. Auf der Austrittsöffnung (15) der Karpule (10) ist ein Septum (16) aus Silikonkunststoff mit einem dünnen Aluminiumblech (17) aufgeklemmt.

Figur 2b zeigt eine Durchstechungsvorrichtung (20). Sie besitzt einen zylindrischen Mantel (21) aus Glas. Ein Ende dieses Mantels (21) ist durch einen Kunststoffpropfen (22) verschlossen, in dem eine Kanüle (23) steckt, deren beide Enden angeschrägt sind. Die Karpule (10) kann mit einer Durchstechungsvorrichtung (20) entsiegelt werden. Zu diesem Zweck wird die Karpule (10) in die Durchstechungsvorrichtung (20) geschoben, wobei die in das Innere des Mantels (21) ragende Spitze der Kanüle (23) das Septum (16) durchsticht. Durch Bewegung des Kolbens (12) kann nun Flüssigkeit aus der Karpule abgegeben werden.

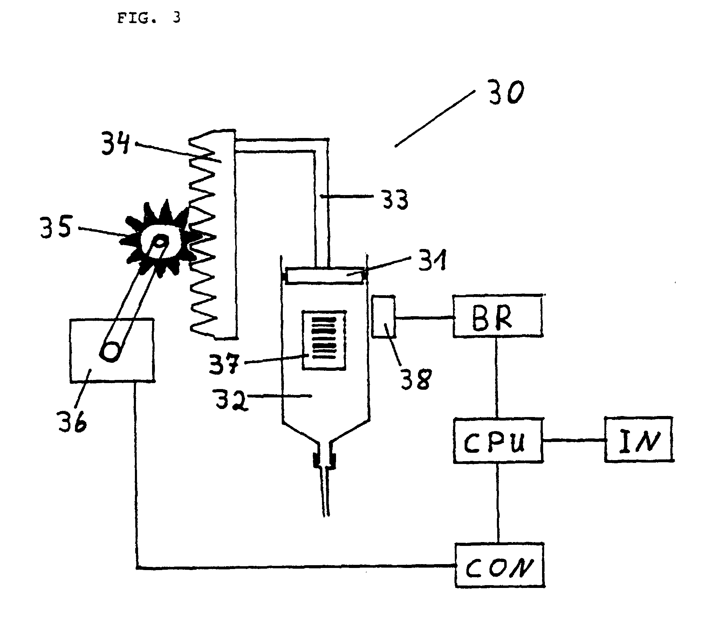

Figur 3 zeigt schematisch ein System (30) zur Dosierung von Flüssigkeiten. Der Kolben (31) einer Dosierungsvorrichtung (32) ist mechanisch mit dem Hebel (33) verbunden, der durch eine Zahnstange (34) bewegt werden kann. Die Zahnstange (34) ihrerseits wird durch ein Zahnrad (35) bewegt, das von einem Motor (36) angetrieben wird. Bei der Installation der Dosierungsvorrichtung (32) im System (30) wird der Strichcode (37) der Dosierungsvorrichtung (32) an einem Sensor (38) vorbeibewegt. Die vom Sensor (38) abgegebenen Impulse werden von einem Strichcodelesegerät (BR) verarbeitet und an eine elektronische Einheit (CPU) zur Berechnung weitergegeben. Die Einheit (CPU) erhält außerdem Signale von einer Eingabetastatur (IN). In der Einheit zur Berechnung (CPU) werden die eingegangenen Signale verarbeitet und ein Dosierhub berechnet. Die Einheit (CPU) gibt ihrerseits Signale an einen Konverter (CON) weiter, der den Motor (36) ansteuert.

## Bezugszeichenliste

(1) Dosiervorrichtung
(2) Spritzenmantel
(3) Kolben
(4) Stempel
(5) O-Ring
(6) Strichcode
(7) Kolbenöffnung
(8) Austrittsöffnung
(9) -
(10) Karpule
(11) Spritzenmantel
(12) Kolben
(13) Lippe zur Abdichtung
(14) Strichcode
(15) Austrittsöffnung
(16) Septum
(17) Aluminiumblech
(18) -
(19) -
(20) Durchstechungsvorrichtung
(21) zylindrischer Mantel
(22) Kunststoffpropfen
(23) Kanüle
(24) -
(25) -
(26) -
(27) -
(28) -
(29) -
(30) System zur Dosierung von Flüssigkeiten
(31) Kolben
(32) Dosierungsvorrichtung
(33) Hebel
(34) Zahnstange
(35) Zahnrad
(36) Motor
(37) Strichcode
(38) Sensor

BR: Strichcodelesegerät
CPU: elektronische Einheit
IN: Eingabetastatur
CON: Konverter

## Patentansprüche

1. System (30) zur Dosierung von Flüssigkeiten, beinhaltend

   - eine Dosiervorrichtung (32) mit einem Spritzenkörper, der einen über die Länge konstanten Innendurchmesser (d) besitzt, und einen darin dichtend laufenden Kolben (31),

   - einen Datenträger (6, 14, 37), der Daten, die für den individuellen Innendurchmesser (d) des Spritzenkörpers charakteristisch sind, und die für den individuellen Spritzenkörper bestimmt wurden, speichert,

   - eine Vorrichtung (33, 34, 35, 36) zur Bewegung des Kolbens (31) in dem Spritzenkörper,

   - ein Lesegerät zum Einlesen der für den individuellen Innendurchmesser (d) des Spritzenkörpers charakteristischen Daten

- eine Vorrichtung zum Einlesen von Daten für eine durchzuführende Dosierung,

- eine elektronische Einheit (CPU) zur Berechnung eines Dosierhubes (h) des Kolbens unter Berücksichtigung der Daten, die für den individuellen Innendurchmesser (d) charakteristisch sind und der Daten für eine durchzuführende Dosierung.

2. System (30) nach Anspruch 2, bei dem die Daten für den Innendurchmesser (d) des Spritzenkörpers in Form eines Balkencodes vorliegen.

3. System (30) nach Anspruch 1, bei dem die Daten für den Innendurchmesser (d) des Spritzenkörpers auf einem Magnetstreifen oder einem Speicherchip vorliegen.

4. System (30) gemäß einem der Ansprüche 1 bis 3, **dadurch gekennzeichnet, daß** die Daten, die für den Innendurchmesser der Dosiervorrichtung charakteristisch sind, auf der Dosiervorrichtung (32) angebracht sind.

5. System (30) gemäß einem der Ansprüche 1 bis 3, **dadurch gekennzeichnet, daß** die Daten, die für den Innendurchmesser der Dosiervorrichtung charakteristisch sind, in einer dem Lesegerät zuzuführenden Form vorliegen, die von der Dosiervorrichtung (32) räumlich getrennt ist

6. System (30) gemäß Anspruch 1, bei dem die Dosiervorrichtung (32) mit Flüssigkeit gefüllt und versiegelt ist.

7. System (30) gemäß Anspruch 6, das eine Vorrichtung zum Aufbrechen der Versiegelung der Dosiervorrichtung (32) besitzt.

8. System gemäß Anspruch 1, bei dem die elektronisch Einheit (CPU) zur Berechnung des Dosierhubes (h) des Kolbens die Formel

$$h = 4 V / (\pi d^2)$$

verwendet.

9. System (30) nach Anspruch 1 mit einer Einwegdosiervorrichtung.

10. Verfahren zur Dosierung von Flüssigkeiten, das folgende Schritte beinhaltet:

- Bereitstellen einer Dosiervorrichtung (32) mit einem Spritzenkörper, der einen über die Länge konstanten Innendurchmesser (d) besitzt, in dem ein Kolben (31) dichtend läuft,

- Bereitstellen eines Datenträgers (6, 14, 37), der Daten, die für den individuellen Innendurchmesser (d) des Spritzenkörpers charakteristisch sind und die für den individuellen Spritzenkörper bestimmt wurden, gespeichert hat,

- Bereitstellen einer Vorrichtung (33, 34, 35, 36) zur Bewegung des Kolbens (31) in dem Spritzenkörper,

- Einlesen der für den Innendurchmesser (d) eines individuellen Spritzenkörpers charakteristischen Daten,

- Einlesen von Daten für eine durchzuführende Dosierung,

- Berechnung eines Dosierhubes (h) des Kolbens unter Berücksichtigung der Daten, die für den Innendurchmesser (d) spezifisch sind und der Daten für eine durchzuführende Dosierung,

- Ausführen des Dosierhubes (h)

11. Verfahren gemäß Anspruch 10, bei dem die dosierte Flüssigkeit eine Reagenzlösung ist,

12. Verfahren gemäß Anspruch 10, bei dem die Dosiervorrichtung nach Entleerung verworfen wird.

13. Verfahren gemäß Anspruch 10, bei dem der Abgabe des zu dosierenden Volumens ein Verfahrensschritt zur Entsiegelung der Dosiervorrichtung vorangeht.

14. Verfahren gemäß Anspruch 10, bei dem die Daten für den Innendurchmesser (d) des individuellen Spritzenkörpers und des Inhaltes der Dosiervorrichtung einmalig nach Installation der Dosiervorrichtung innerhalb des Systems ermittelt werden.

## Claims

1. System (30) for dosing liquids comprising

- a dosing device (32) having a syringe body which has a constant inside diameter (d) over its length and a plunger (31) which forms a seal when it moves within the syringe body,

- a data carrier (6, 14, 37) which stores data that are characteristic for the individual inside diameter (d) of the syringy body and which have been determined for the individual syringe body,

- a device (33, 34, 35, 36) for moving the plunger (31) in the syringe body,

- a reading instrument for reading in characteristic data for the individual inside diameter (d) of the syringe body,

- a device for reading in data for a dosing that is to be carried out

- an electronic unit (CPU) for calculating a metering stroke (h) of the plunger taking into consideration the data that are characteristic for the individual inside diameter (d) and the data for a dosing to be carried out.

2. System (30) as claimed in claim 1, in which the data for the inside diameter (d) of the syringe body are present in the form of a bar code.

3. System (30) as claimed in claim 1, in which the data for the inside diameter (d) of the syringe body are present on a magnetic strip or a storage chip.

4. System (30) as claimed in one of the claims 1 to 3, **characterized in that** the data which are characteristic for the inside diameter of the dosing device are attached to the dosing device (32).

5. System (30) as claimed in one of the claims 1 to 3, **characterized in that** the data which are characteristic for the inside diameter of the dosing device are present in a form which can be conveyed to the reading instrument and are spatially separated from the dosing device (32).

6. System (30) as claimed in claim 1, **characterized in that** the dosing device (32) is filled with liquid and sealed.

7. System (30) as claimed in claim 6, **characterized in that** it has a device to break open the seal of a dosing device (32).

8. System as claimed in claim 1, in which the electronic unit (CPU) uses the formula

$$h = 4V/(\pi d^2)$$

to calculate the metering stroke (h) of the plunger.

9. System (30) as claimed in claim 1 with a disposable dosing device.

10. Process for dosing liquids comprising the following steps:

- providing a dosing device (32) having a syringe body which has a constant inside diameter (d) over its length and a plunger (31) which forms a seal when it moves within the syringe body,

- providing a data carrier (6, 14, 37) which has stored data that are characteristic for the individual inside diameter (d) of the syringy body and which have been determined for the individual syringe body,

- providing a device (33, 34, 35, 36) for moving the plunger (31) in the syringe body,

- reading in characteristic data for the inside diameter (d) of an individual syringe body,

- reading in data for a dosing that is to be carried out,

- calculating a metering stroke (h) of the plunger taking into consideration the data that are characteristic for the inside diameter (d) and the data for a dosing to be carried out

- performing the metering stroke (h).

11. Process as claimed in claim 10, in which the metered liquid is a reagent solution.

12. Process as claimed in claim 10, in which the dosing device is discarded after being emptied.

13. Process as claimed in claim 10, in which a process step for unsealing the dosing device proceeds the dispensing of the volume to be dosed.

14. Process as claimed in claim 10, in which the data for the inside diameter (d) of the individual syringe body and the contents of the dosing device are determined once after installing the dosing device within the system.

**Revendications**

1. Système (30) pour le dosage de liquides, contenant

- un dispositif de dosage (32) avec un corps de seringue qui possède un diamètre interne (d) constant sur toute sa longueur et un piston (31) qui s'y déplace de manière étanche,

- un support de données (6, 14, 37) qui mémorise des données qui sont caractéristiques pour le diamètre interne individuel (d) du corps de seringue et qui ont été déterminées pour le corps de seringue individuel,

- un dispositif (33, 34, 35, 36) pour le déplacement du piston (31) dans le corps de seringue,

- un appareil de lecture pour la lecture des données caractéristiques pour le diamètre interne individuel (d) du corps de seringue,

- un dispositif pour la lecture de données pour un dosage qui doit être mis en oeuvre,

- une unité électronique (CPU) pour le calcul d'une course de dosage (h) du piston en prenant en compte les données qui sont caractéristiques pour le diamètre interne individuel (d), ainsi que les données pour un dosage à mettre en oeuvre.

2. Système (30) selon la revendication 1, dans lequel les données pour le diamètre interne (d) du corps de seringue sont présentes sous la forme d'un code-barres.

3. Système (30) selon la revendication 1, dans lequel les données pour le diamètre interne (d) du corps de seringue sont présentes sur une bande magnétique ou sur une puce à mémoire.

4. Système (30) selon l'une quelconque des revendications 1 à 3, **caractérisé en ce que** les données, qui sont caractéristiques pour le diamètre interne du dispositif de dosage, sont appliquées sur le dispositif de dosage (32).

5. Système (30) selon l'une quelconque des revendications 1 à 3, **caractérisé en ce que** les données, qui sont caractéristiques pour le diamètre interne du dispositif de dosage, sont présentes sous une forme destinée à être acheminé à l'appareil de lecture, qui est séparée, du point de vue spatial, du dispositif de dosage (32).

6. Système (30) selon la revendication 1, dans lequel le dispositif de dosage (32) est rempli avec un liquide et est fermé de manière hermétique.

7. Système (30) selon la revendication 6, qui possède un dispositif pour rompre la fermeture hermétique du dispositif de dosage (32).

8. Système selon la revendication 1, dans lequel l'unité électronique (CPU) utilise, pour le calcul de la course de dosage (h) du piston, la formule :

$$h = 4\ V/(\pi d^2).$$

9. Système (30) selon la revendication 1, comprenant un dispositif de dosage à usage unique.

10. Procédé pour le dosage de liquides, qui comprend les étapes ci-après consistant à :

- mettre à disposition un dispositif de dosage (32) avec un corps de seringue qui possède un diamètre interne (d) constant sur toute sa longueur, dans lequel se déplace un piston (31) de manière étanche,

- mettre à disposition un support de données (6, 14, 37) qui a en mémoire des données qui sont caractéristiques pour le diamètre interne individuel (d) du corps de seringue et qui ont été déterminées pour le corps de seringue individuel,

- mettre à disposition un dispositif (33, 34, 35, 36) pour le déplacement du piston (31) dans le corps de seringue,

- lire des données caractéristiques pour le diamètre interne (d) d'un corps de seringue individuel,

- lire les données pour un dosage qui doit être mis en oeuvre,

- calculer une course de dosage (h) du piston en prenant en compte les données qui sont spécifiques pour le diamètre interne (d), ainsi que les données pour un dosage à mettre en oeuvre,

- mettre en oeuvre la course de dosage (h).

11. Procédé selon la revendication 10, dans lequel le liquide dosé est une solution de réactif.

12. Procédé selon la revendication 10, dans lequel on jette le dispositif de dosage une fois qu'il est vide.

13. Procédé selon la revendication 10, dans lequel la libération du volume à doser est précédée par une étape opératoire destinée à rompre la fermeture hermétique du dispositif de dosage.

14. Procédé selon la revendication 10, dans lequel les données du diamètre interne (d) du corps de seringue individuel moulé et du contenu du dispositif de dosage sont soumises à une détermination unique après le montage du dispositif de dosage au sein du système.

EP 0 618 426 B1

FIG. 1

FIG. 2

a)

b)

10

FIG. 3